# EUROPEAN PATENT APPLICATION

(11) **EP 4 478 376 A1**
(43) Date of publication of application: **18.12.2024**
(21) Application number: 24181589.3
(22) Date of filing: 12.06.2024
(51) Int. Cl.: G16H 40/63, G16H 40/67

(54) **AUTOMATIC CONFIGURATION FOR INFUSION SYSTEM**

(30) Priority: 13.06.2023 US 202363507760 P
(71) Applicant: Fresenius Kabi USA, LLC, Lake Zurich, IL 60047 (US)
(72) Inventor: PASTORE, Christopher, Lake Zurich, 60047 (US); MICHAELS, Ian, Lake Zurich, 60047 (US)
(74) Representative: Maikowski & Ninnemann Patentanwälte Partnerschaft mbB

(57) **Abstract**

An infusion system includes an infusion endpoint configured to deliver a medical fluid and a broadcaster configured to communicate with the infusion endpoint. The infusion endpoint includes a controller and a communication module configured to communicate using first and second communication mediums. The broadcaster includes a wireless transmitter and a computing device configured to provide a broadcasting utility. The broadcasting utility is configured to prepare a message for broadcast by the wireless transmitter, and the communication module is configured to receive the message via a wireless link using the first communication medium. The controller is configured to determine one or more configuration parameters from the message, connect to a network according to the configuration parameters and disable the first communication medium when connected to the network. The communication module is configured to communicate with the network via a network link using the second communication medium.

## Description

### TECHNICAL FIELD

The present disclosure is generally directed to infusion systems and methods. More particularly, the present disclosure is directed to the automatic configuration of one or more infusion endpoints of an infusion system at a customer site.

### BACKGROUND

An infusion system installed at a customer site includes a number of infusion devices having fluid delivery pumps for delivering a medical fluid to a patient. The infusion devices may be connected over a communication network such that the individual devices can send and receive information to a server or other computing device. In this manner, the infusion devices can receive operating parameters or instructions to control the fluid delivery pumps to perform an infusion procedure. The infusion devices may also provide operating information regarding performance of the infusion procedure to the server or other computing device.

Currently, to set up the infusion system at the customer site, the infusion devices are separately and individually configured to communicate over the network. For example, a current infusion device may be configured by entering configuration settings via a touchscreen display on the device. After entering the configuration settings, a technician or other user provides a start instruction to the device via the touchscreen display or other input. In response to receiving the start instruction, the device may perform configuration steps using the configuration settings to connect the device to the communication network for network communications. Thus, in current systems, the infusion devices are configured for network communication one at a time by manual input of the configuration settings and subsequently providing a respective start instruction to the devices. Accordingly, setting up the infusion system may be time and labor intensive, and further, may be subject to human error as configuration settings are individually entered into the infusion devices.

### SUMMARY

According to an aspect, an infusion system includes an infusion endpoint configured to deliver a medical fluid and a broadcaster configured to broadcast one or more messages to the infusion endpoint. The infusion endpoint includes a controller configured to control the infusion endpoint according to one or more configuration parameters and a communication module coupled to the controller and configured to communicate using a first communication medium and a second communication medium. The broadcaster includes a wireless transmitter in communication with the communication module via a wireless link using the first communication medium, and a computing device configured to provide a broadcasting utility. The broadcasting utility is configured to prepare the message for broadcast by the wireless transmitter and received by the communication module, the message including data that is usable by the controller to determine at least one of the one or more configuration parameters. The controller is configured to control the infusion endpoint to connect to a communication network according to the one or more configuration parameters and the communication module is configured to communicate with the communication network via a network link using the second communication medium. The controller is configured to disable the first communication medium when the communication module communicates using the second communication medium.

According to another aspect, a method of configuring one or more infusion endpoints in an infusion system is provided. Each infusion endpoint includes a controller, a communication module, one or more pumps for delivering a medical fluid, and an endpoint user interface, wherein the communication module is configured to communicate using a first communication medium and a second communication medium. The method includes receiving, by the communication module, via a wireless link using the first communication medium, a broadcast message including data relating to one or more configuration parameters, determining, by the controller, at least one of the one or more configuration parameters using the data in the message, automatically completing, by the controller, a configuration step using at least one of the one or more configuration parameters, wherein the configuration step is a configuration step for connecting to a communication network, establishing a secure connection with the communication network via a network link using the second communication medium, and disabling, by the controller, communications using the first communication medium when the communication module communicates with the communication network using the second communication medium. Its to be understood that the method described refers to a configuration of devices, here pumps, and thus is to be seen as a non-therapeutic method.

### BRIEF DESCRIPTION OF THE DRAWINGS

The disclosure will be more fully understood from the following description taken in conjunction with the accompanying drawings. Some of the figures may have been simplified for the purpose of more clearly showing the systems and methods described herein. Any such simplified depictions in some figures are not necessarily indicative of the presence or absence of particular elements in any of the exemplary embodiments, except as may be explicitly delineated in the corresponding written description. None of the drawings is necessarily to scale.
Fig. 1 is a schematic diagram of an embodiment of an infusion system having a broadcaster and an infusion endpoint;
Fig. 2 is a schematic diagram of the infusion system of Fig. 1 having a plurality of infusion endpoints;
Fig. 3 is a schematic diagram of the infusion system of Fig. 2, wherein the broadcaster communicates with a single infusion endpoint to which the message is sent;
Fig. 4 is a diagram of the infusion system of Fig. 1 showing a broadcast range of the broadcaster;
Fig. 5 is a schematic diagram of an embodiment of a broadcaster for use in the infusion system of Fig. 1;
Fig. 6 is a schematic diagram of an embodiment of an infusion endpoint for use in the infusion system of Fig. 1;
Fig. 7 is a schematic diagram of an embodiment of a communication module for use with the infusion endpoint of Fig. 6;
Fig. 8 is a schematic diagram of an embodiment of an endpoint user interface for use with the infusion endpoint of Fig. 6;
Fig. 9 is a schematic diagram of an embodiment of a controller for use with the infusion endpoint of Fig. 6;
Fig. 10 is a schematic diagram of a broadcaster and an infusion endpoint for use in the infusion system of Fig. 1, showing a message transmitted via a wireless link using a first communication medium;
Fig. 11 is a diagram showing an example of the message of Fig. 10 containing data relating to one or more configuration parameters;
Fig. 12 is a schematic view of an embodiment of the infusion system of Fig. 1 in which an infusion endpoint is connected to a communication network; and
Fig. 13 is a block diagram showing an embodiment of a method for automatically configuring an infusion endpoint in an infusion system.

### DETAILED DESCRIPTION OF VARIOUS EMBODIMENTS

A detailed description of an infusion endpoints and methods in accordance with the present disclosure is set forth below. It should be understood that the description below of specific devices and methods is intended to be exemplary, and not exhaustive of all possible variations or applications. Thus, the scope of the disclosure is not intended to be limiting and should be understood to encompass all variations or embodiments that would occur to persons of ordinary skill.

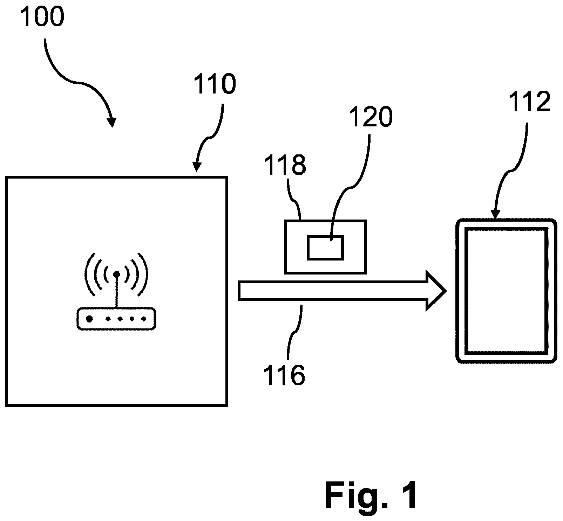

Referring to Figs. 1-3, embodiments of an infusion system 100 include a broadcaster 110 and an infusion endpoint 112. The broadcaster 110 is configured to communicate with the infusion endpoint 112 over a wireless link 116 using a first communication medium such that the infusion endpoint 112 may receive one or more messages 118 broadcast by the broadcaster 110 via the wireless link 116. The message 118 includes data relating to one or more configuration parameters 120 for the endpoint 112. The endpoint 112 is configured to receive the one or more messages 118 and determine the one or more configuration parameters 120 from the data in the message(s) 118. The endpoint 112 may populate one or more data fields with corresponding configuration parameters 120.

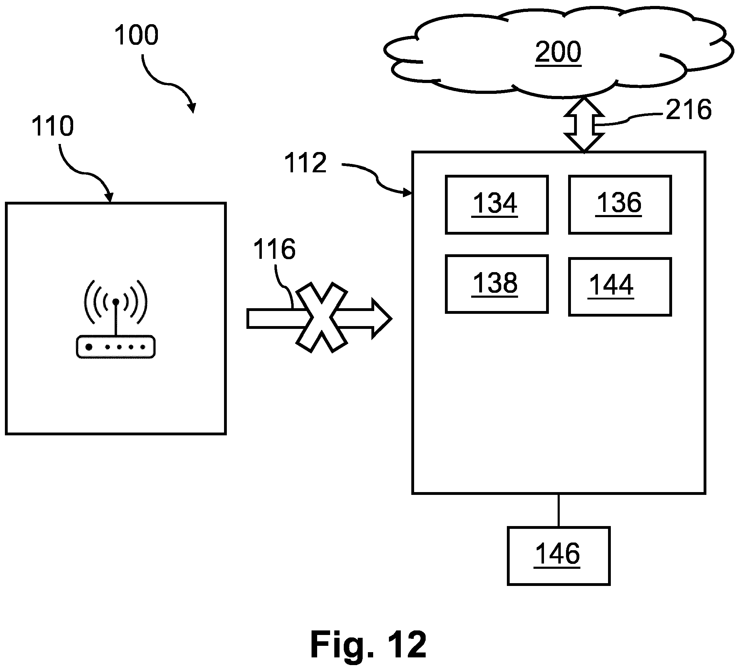

The one or more configuration parameters 120 may include network parameters for configuring the infusion endpoint 112 to communicate over a communication network 200 (see Fig. 12). The infusion endpoint 112 may be configured to automatically perform one or more configuration steps to connect to the communication network 200 in response to determining the one or more configuration parameters 120.

The infusion endpoint 112 of the present embodiments may be implemented as an infusion device configured to deliver a medical fluid, such as a nutritional fluid, a biological fluid and/or a therapeutic drug to a patient. The infusion endpoint 112 may be located at a medical facility (sometimes referred to as a "customer site") where medical infusion procedures are performed. The infusion endpoint 112 may be arranged in a patient treatment room, and in the manner described above, can be configured to connect to a communication network according to the configuration parameters 120. The infusion endpoint 112 may then communicate with the communication network 200 via a network link 216 (Fig. 12) using a second communication medium. The infusion endpoint 112 may receive operating parameters and/or other information relating to the performance of a selected infusion procedure via the network link 216 and operate in accordance with the received operating parameters and/or other information.

Thus, according to the present embodiments, an infusion endpoint 112 may be configured remotely by way of the message(s) 118 received from the broadcaster 110. Accordingly, it is not necessary for a technician to manually configure the infusion endpoint 112. That is, by populating the one or more data fields of one or more infusion endpoints 112 with corresponding configuration parameters 120 received via the broadcasted message(s) 118, manual entry of the configuration parameters 120 via a user interface of the infusion endpoint 112 may be avoided. In this manner, errors associated with manual input of the configuration parameters at individual endpoints 112 may be limited or avoided. In addition, multiple infusion endpoints 112 may be configured according to the configuration parameters 120 in response to the broadcaster 110 broadcasting a single broadcast message 118 or sequence of broadcast messages 118. Accordingly, the infusion endpoint(s) 112 may be configured in a shorter time using the broadcaster 110 compared to manual input of the configuration parameters 120 at each infusion endpoint 112. Further still, the infusion endpoint 112 may automatically initiate one or more configuration steps to connect to the communication network in response to receiving one or more of the configuration parameters 120. Thus, the infusion endpoint 112 may connect to the communication network 200 by receiving the message(s) 118 in a broadcast without a technician manually inputting network configuration parameters or a subsequent start instruction to the endpoint 112.

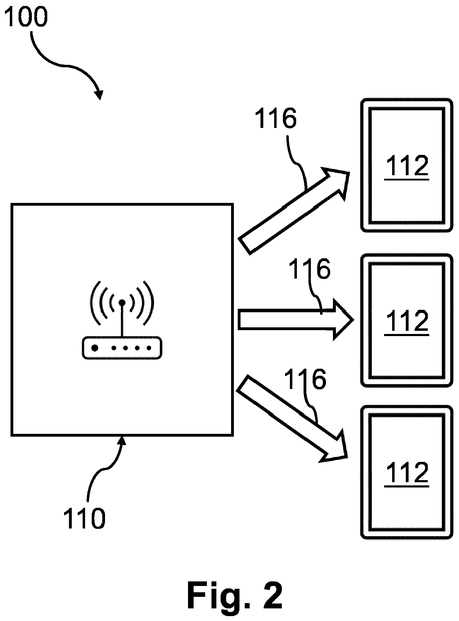

With reference to Fig. 2, the infusion system 100 may include a plurality of the infusion endpoints 112 and the broadcaster 110 may be configured to communicate with each of the infusion endpoints 112 via one or more of the wireless links 116 using the first communication medium. For example, the broadcaster 110 may transmit a message 118 (see Fig. 1) to each infusion endpoint 112 via respective wireless links 116 using the first communication medium.

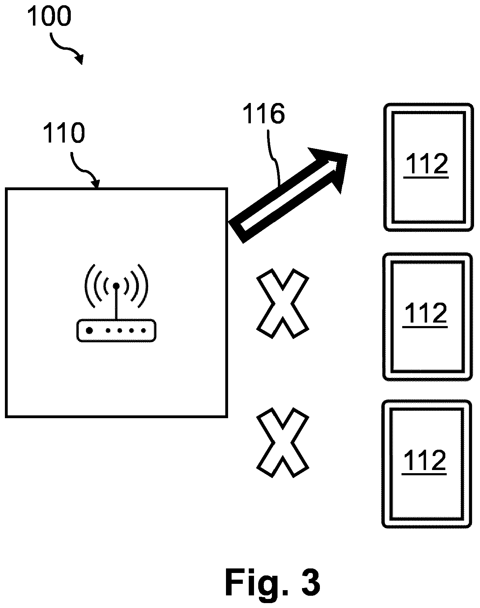

Now referring to Fig. 3, the infusion system 100 may include the plurality of infusion endpoints 112 and the broadcaster 110 may be configured to communicate the message 118 (see Fig. 1) to only one of the infusion endpoints 112. Thus, the wireless link 116 using the first communication medium may be provided between the broadcaster 110 and one of the infusion endpoints 112. In one example, the broadcaster 110 selects the infusion endpoint 112 to which the message 118 is transmitted according to indicia associated with the infusion endpoint 112. Non-limiting examples of such indicia may include a serial number, endpoint name, model and/or other identifying information associated with the infusion endpoint 112. In some examples, the indicia, such as a serial number, for each infusion endpoint 112 is associated with the medical facility wherein the infusion endpoint 112 is located. Alternatively, or in addition, a selected infusion endpoint 112 to be configured may be operated in a mode for receiving a message 118 broadcast by the broadcaster 110. For example, the selected infusion endpoint 112 may be operated in a mode to enable communication using the first communication medium, and thus, may listen for the message 118 broadcasted by the broadcaster 110.

Accordingly, in the examples above, one or more of the infusion endpoints 112, and in one example, all the infusion endpoints 112, may be remotely configured, simultaneously in some instances, when setting up the infusion endpoints 112 for network communications at the medical facility. For example, the infusion endpoints 112 may be remotely configured for communication on a selected communication network 200 at the medical facility by providing the one or more configuration parameters to the endpoints 112 in the manner described above.

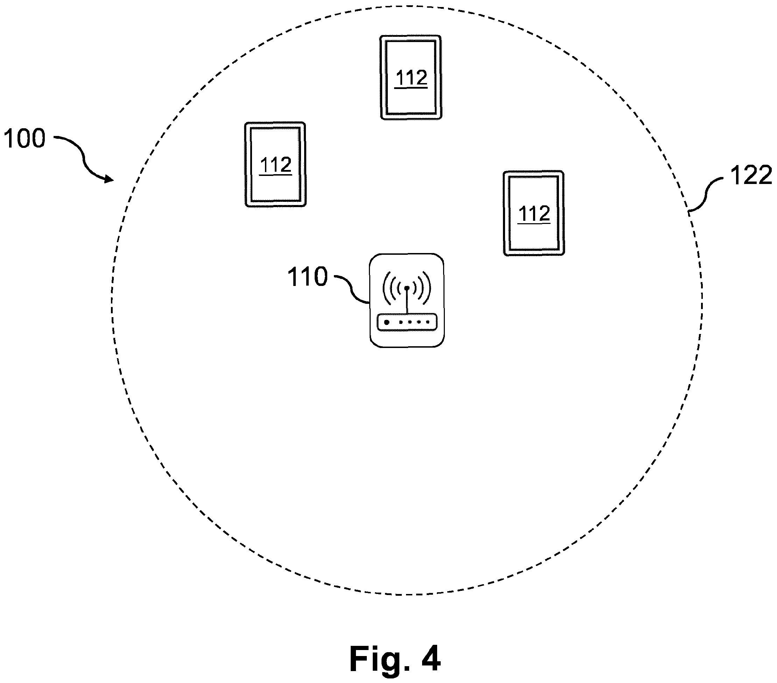

As shown in Fig. 4, the broadcaster 110 is configured to broadcast the one or more messages 118 (Fig. 1) over a broadcast range 122. The broadcast range 122 may vary depending on, for example, the first communication medium 116, transmitting power and/or physical structures at the customer site.

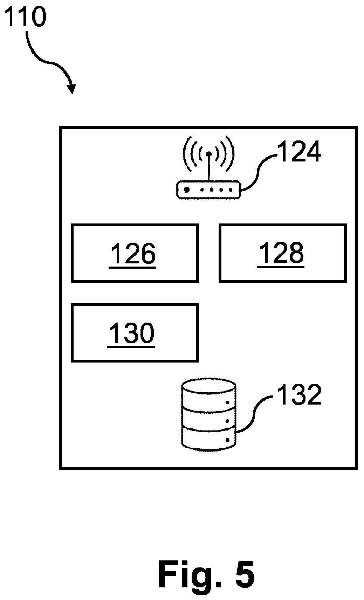

Referring now to Fig. 5, the broadcaster 110 generally includes a wireless transmitter 124 and a computing device 126 configured to provide a broadcasting utility 128. The broadcasting utility 128 is configured to prepare the message 118 and the wireless transmitter 124 is configured to broadcast the message 118 using the first communication medium. In one embodiment, the computing device 126, via the broadcasting utility 128, is configured to prepare the message 118 by encoding the data relating to the one or more configuration parameters 120.

The computing device 126 may be coupled to a broadcaster input device 130 configured as a user interface. The computing device 126 is configured to receive at least one of the one or more configuration parameters 120 from the broadcaster input device 130 and provide the at least one of the one or more configuration parameters 120 to the broadcasting utility 128. The broadcaster input device 130 may be integrated with the broadcaster 110 as shown in the example of Fig. 5, or alternatively, may be connected to the broadcaster 110 by a suitable connection facilitating electronic communication between the broadcaster input device 130 and the computing device 126.

The computing device 126 may be coupled to a database server 132 and may be configured to receive at least one of the one or more configuration parameters 120 from the database server 132 and provide the at least one of the one or more configuration parameters 120 to the broadcasting utility 128. Thus, in the present embodiments, at least one of the one or more configuration parameters 120 may be provided to the computing device 126, for example, by user input at the broadcaster input device 130 and/or by retrieving one or more stored configuration parameters 120 from the database server 132. The database server 132 may be integrated with the broadcaster 110 as shown in the example of Fig. 5, or alternatively, may be connected to the broadcaster 110 by a suitable connection facilitating electronic communication between the database server 132 and the computing device 126.

The computing device 126 of the present embodiments may include, for example, a processing unit and one or more memory devices coupled to the processing unit. The processing unit is configured to execute program instructions and in response to executing the program instructions, perform one or more functions according to the program instructions. In a non-limiting example, the processing unit may include a microprocessor. However, other processors are also envisioned. The one or more memory devices may include any suitable data storage mechanism or system, such as a random access memory (RAM), read only memory (ROM), disk drive, solid state memory drive, optical disc drive and the like, including various combinations of such data storage mechanisms or systems. The one or more memory devices may also include, for example, a non-transitory computer-readable storage medium configured to store and allow access to the program instructions, code and/or other data. The computing device 126 may also include a communication module to facilitate communication with one or more electronic devices.

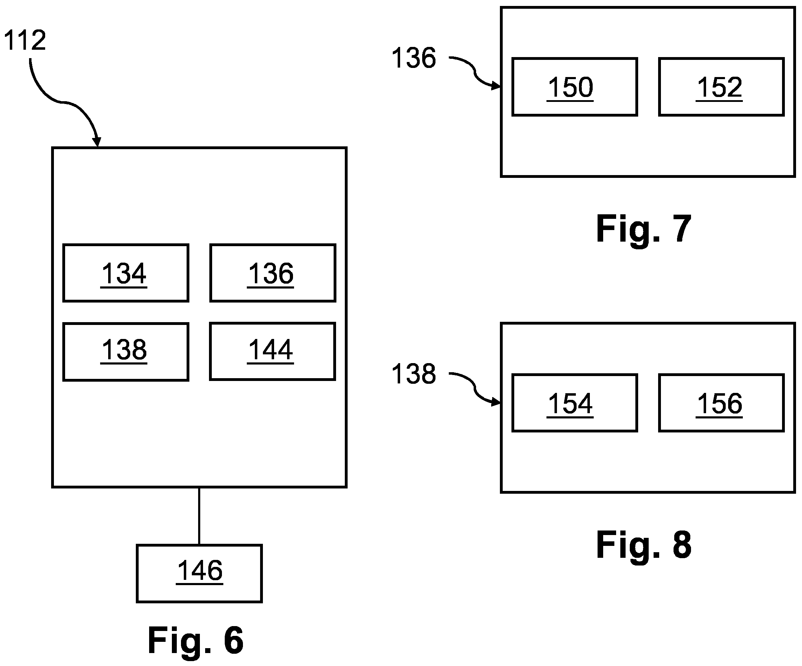

With reference to Fig. 6, the infusion endpoint 112 includes a controller 134 and a communication module 136 coupled to the controller 134. The communication module 136 is configured to communicate using at least two different communication mediums, for example the first communication medium and the second communication medium. For example, the infusion endpoint 112 may be configured to receive the one or more broadcast messages 118 via the wireless link 116 using the first communication medium and to communicate with the network 200 via the network link 216 using the second communication medium.

In one embodiment, the communication module 136 includes one or more communication devices configured to facilitate wired and/or wireless communication. The one or more communication devices may include one or more transmitters, receivers and/or transceivers which may be configured to transmit and/or receive carrier signals using a selected communication medium. In various examples, the one or more communication devices include one or more wireless communication devices configured to accommodate wireless communication.

As shown in Fig. 7, in one embodiment, the communication module 136 includes a first communication device 150 configured to communicate using the first communication medium 116 and a second communication device 152 configured to communicate using the second communication medium 216. The first communication device 150 may include at least a wireless receiver configured to receive the broadcasted message 118. The second communication device 152 may include a wireless transmitter, a wireless receiver and/or a wireless transceiver configured to communicate with the communication network 200 (Fig. 12). Alternatively, or in addition, the second communication device 152 may include one or more devices to facilitate wired communication with the communication network 200.

The controller 134 may be coupled to an endpoint user interface 138 configured to receive information from and output information to the user. As shown in Fig. 8, the endpoint user interface 138 may include an endpoint input device 154 and/or an endpoint output device 156. The endpoint user interface 138 may be integrated with the infusion endpoint 112 as shown in the example of Fig. 6, or alternatively, may be connected to the infusion endpoint 112 by a suitable connection facilitating electronic communication between the endpoint user interface 138 and the controller 134.

In non-limiting examples, the endpoint input device 154 may include one or more of a touchscreen display, keypad, keyboard, pointer device, buttons, knobs, levers, optical readers / scanner (e.g., barcode and/or QR code scanner), biometric sensors, microphones and/or any other suitable input device configured to allow a user to input information to the infusion endpoint 112. The endpoint input device 154 may include various combinations of input devices as well. In one example, the endpoint input device 154 may be configured to receive at least one other configuration parameter from a user relating to a network parameter for configuring the endpoint 112 to communicate over the network 200. In some examples, the endpoint input device 154 may be configured to receive one or more operating parameters or other information relating to performance of an infusion procedure as well.

In non-limiting examples, the endpoint output device 156 may include one or more of a display (such as the touchscreen display), indicator lights, audio speakers and/or any other suitable output device configured to provide information and/or alerts to the user. The output device 156 may include various combinations of output devices as well. The endpoint output device 156 may be configured to output information to a user including, for example, configuration information of the infusion endpoint 112, procedure information relating to an infusion procedure, and/or various other information such a status indicator, alarm, alert, warning, instructions and the like.

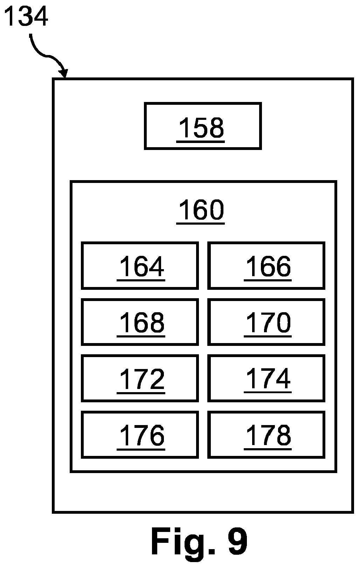

With reference to Fig. 9, the controller 134 may include, for example, at least one processing unit 158 operatively coupled to one or more memory devices 160. The processing unit 158 may include one or more processors, microprocessors and/or other suitable circuitry configured to execute one or more program instructions, and in response to executing the program instructions, perform one or more functions according to the program instructions.

The one or more memory devices 160 may include any suitable data storage mechanism or system, such as a random access memory (RAM), read only memory (ROM), disk drive, solid state memory drive, optical disc drive and the like, including various combinations of such data storage mechanisms or systems. The one or more memory devices 160 may also include, for example, a non-transitory computer-readable storage medium configured to store and allow access to the program instructions, code and/or other data.

In some examples, the one or more memory devices 160 may be configured to store data in one or more data fields, for example, data fields 164, 166, 168, 170, 172, 174, 176, 178 shown in Fig. 9. It will be appreciated that the present disclosure is not limited to the number of data fields illustrated in Fig. 9, and that the number of data fields may vary as needed, for example, to include more data fields or fewer data fields than shown. In one example, the data fields 164, 166, 168, 170, 172, 174, 176, 178 may be populated with corresponding configuration parameters 120 determined from the data in message 118. It will be appreciated that the memory device(s) 160 may also include additional data fields configured to be populated with additional or different parameters (e.g., operating parameters) or other information.

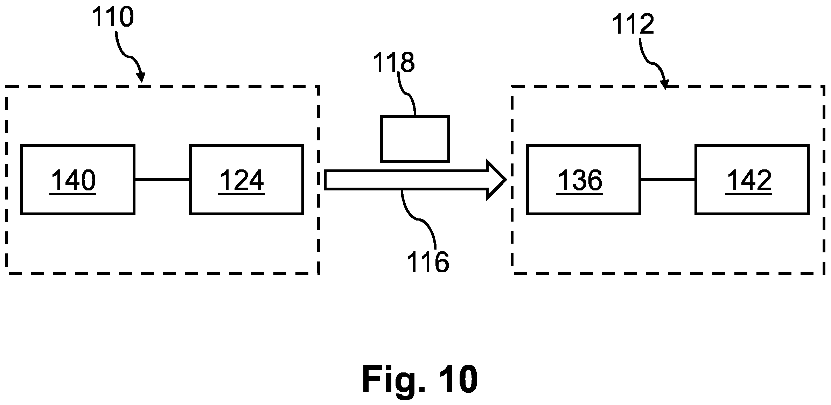

Turning to Fig. 10, the wireless transmitter 124 of the broadcaster 110 may be disposed in communication with the communication module 136 of the infusion endpoint 112 via the wireless link 116. The wireless transmitter 124 may broadcast the message(s) 118 over the wireless link 116 using the first communication medium. Thus, the message 118 broadcasted by the wireless transmitter 124 may be received by the communication module 136 using the first communication medium. The message 118 includes data usable by the controller 134 (see Fig. 6, for example) to determine at least one of the one or more configuration parameters 120. In addition, the data relating to the one or more configuration parameters 120 may be encoded by an encoder 140 of the computing device 126 before being broadcast by the broadcaster 110. That is, the message 118 may be an encoded message. The controller 134 may include a decoder 142 configured to decode the encoded message 118 and determine the at least one of the one or more configuration parameters 120 from the decoded data. In further examples, the data relating to the one or more configuration parameters may be encrypted by the computing device 126 and the controller 134 may be configured to decrypt the encrypted message 118 and determine the at least one of the one or more configuration parameters 120 from the decrypted data.

The wireless link 116 may use any suitable first communication medium for facilitating wireless communication between the broadcaster 110 and the infusion endpoint 112. In various examples, the first communication medium may facilitate relatively short-range wireless communications, generally limited to a selected area of the customer site, such as a treatment room. In various examples, the first communication medium may facilitate short-range wireless communications using one or more known, suitable radio, optical wireless, acoustical and/or magnetic (e.g., magneto-inductive) communication technology. In some examples, the communication technology may be a low-energy communication technology. Various, non-limiting examples of suitable short-range wireless communication technologies used as the first communication medium may include Bluetooth, Bluetooth Low Energy, Wi-Fi, ZigBee (Low-Power Wireless Personal Area Network), Ultra-Wideband (UWB), Infrared (IR), Near-Field Communication (NFC), and/or Frequency-Shift Keying (FSK) communication technologies, including audio FSK communication technologies. Accordingly, the wireless transmitter 124 and the communication module 136 may be configured to communicate using one or more short-range wireless communication technologies, such as those listed above.

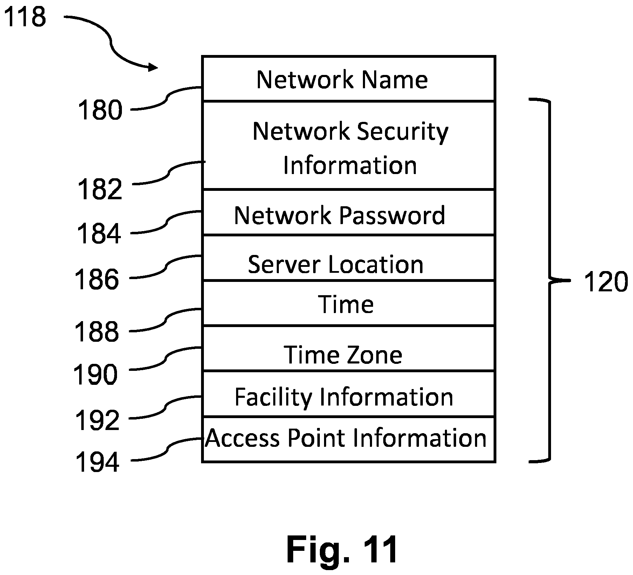

Fig. 11 shows an example of the message 118 including the data relating to the one or more configuration parameters 120. The one or more configuration parameters 120 may include various network setting information, such as, but not limited to, network name information 180 of a network to which the infusion endpoint 112 is to be connected, network security setting information 182 (e.g., WPA2, WPA3, etc.) and/or password information 184 for joining the network 200. Other configuration parameters 120 may include, for example, server location information 186, such as a server address ("url") for a server with which the infusion endpoint 112 may communicate via the network 200, and time 188 and/or time zone information 190. Non-limiting examples of further configuration parameters 120 may include facility information 192 relating to a facility or institution at which the infusion system 100 is installed and/or network access point information 194. It will be appreciated that the one or more configuration parameters 120 may be provided together in a single message 118, or in separate messages 118 containing one or more different configuration parameters 120. The separate message 118 may be broadcast according to a broadcast sequence, in some examples.

Referring again to FIG. 6, the infusion endpoint 112 further includes one or more pump 144 configured to deliver a medical fluid to a patient in a medical infusion procedure. The one or more pump 144 is operated according to various operating parameters corresponding to the selected infusion procedure. The operating parameters for the one or more pump 144 may include, for example, a flow rate, duration, intervals, etc. In a non-limiting example, the one or more pump 144 may include a pneumatically-driven volumetric pump. Other suitable pumps include, for example, peristaltic pumps and diaphragm pumps. The infusion endpoint 112 may be connected to a fluid supply 146 configured to provide the medical fluid to the one or pumps 144 via one or more conduits (not shown). The one or more pumps 144 may be operated to draw the medical fluid from the fluid supply 146 and/or to deliver the medical fluid to a patient. In some examples, the medical fluid may be directed to flow through the one or more pumps 144. Alternatively, or in addition, the one or more pumps 144 may act on the one or more conduits to direct the medical fluid through the conduits at the selected flow rate.

In the embodiments above, the controller 134 uses the one or more configuration parameters 120 to perform a configuration procedure for connecting the infusion endpoint 112 to the communication network 200, as shown in Fig. 12. The controller 134 may be configured to automatically complete one or more configuration steps after the message 118 has been decoded and/or the at least one of the one or more configuration parameters 120 determined. That is, the controller 134 may be configured to control the infusion endpoint 112, for example, via the communication module 136, to connect to the communication network 200 by automatically performing one or more configuration steps for connecting to the network 200 in response to receiving the one or more configuration parameters 120.

As shown in Fig. 12, the infusion endpoint 112 may be connected to the communication network 200 via network link 216 using the second communication medium. In some examples, the controller 134 may be further configured to disable the wireless link 116 to prevent communication using the first communication medium when communications using the second communication medium are enabled, i.e., when the network link 216 is established. For example, the controller 134 may disable communications using the first communication medium after receiving all configuration parameters 120 (in message(s) 118) for connecting to the network 200 or once a connection with the network 200 is confirmed. In this manner, the infusion endpoint 112 may be restricted to communicating using the second communication medium when the first communication medium is disabled such that information received from the network 200 via the second communication link 216 may not be accessed using the first communication medium and vice versa.

The infusion endpoint 112 may receive various types of information from the communication network 200 and transmit various types of information to the communication network 200 via the second communication medium 216. Such information may include access-controlled information to be accessed only by authorized personnel. Such access-controlled information may include, but is not limited to, operating parameters of the pump 144 for a selected infusion procedure for a patient, private / confidential patient information including personal information, medical history, medications, treatments, medical diagnoses, EMR information and the like, medical personnel authorized to treat patients, and medical personnel information.

Accordingly, user and/or device authentication may be required to facilitate communication between the infusion endpoint 112 and the network 200 using the second communication medium. Thus, the infusion endpoint 112 and network 200 may communicate using secure communication protocols, such as, WPA2, HTTPS, as well other known, suitable secure protocols. The second communication medium may be, for example, a wired or wireless communication medium, such as, but not limited to, Wi-Fi or cellular communication. In one example, the infusion endpoint 112 may communicate over wireless local area network (WLAN) at the medical facility and may be connected to an access point (not shown) of the network.

Accordingly, in the embodiments above, an infusion endpoint 112 may be configured to receive one or more messages 118 from the broadcaster 110 via the wireless link 116 using the first communication medium and may be further configured to communicate with the communication network 200 via the network link 216 using the second communication medium 216. The second communication medium 216 may be different than the first communication medium 116. The first communication medium 116 may be used for broadcasting messages which may be received by any device within the broadcast range 122 (Fig. 4) configured to listen for the broadcast messages. The second communication medium 216 may use a secure communication protocol to facilitate secure communications of various information, including access-controlled information, between the network 200 and a selected infusion endpoint 112 connected to the network 200. In some examples, the infusion endpoint 112 may disable the first communication medium 116 when the second communication medium is enabled. That is, the controller 134 may prevent the communication module 136 from communicating using the first communication medium when the second communication medium is enabled.

For example, in the present embodiments, a broadcast device 110 is configured to broadcast one or more messages 118 containing data relating to one or more configuration parameters 120 over the broadcast range 122 using the first communication medium. The broadcast device 110 may be, for example, a laptop or personal computer, tablet, mobile phone or other suitable electronic or portable electronic device configured to broadcast one or more messages 118 using the first communication medium in the manner described above.

Infusion endpoints 112 (or a single infusion endpoint) within the broadcast range 122 may be configured to listen for the broadcast messages 118 and may receive the one or more broadcasted messages 118 via the wireless link 116 using the first communication medium. The infusion endpoint(s) 112 may determine one or more configuration parameters 120 based on the data relating to the one or more configuration parameters 120 received in the broadcast message(s) 118 and populate data fields 164, 166, 168, 170, 172, 174, 176, 178 corresponding to the one or more configuration parameters 120.

The infusion endpoint 112 may be configured to automatically complete one or more configuration steps to connect to the communication network 200 in response to determining the one or more configuration parameters 120. The one or more configuration steps may be considered "preliminary" configuration steps in that such steps are performed to establish a connection between infusion endpoint 112 and the communication network 200. Such configuration steps may include listening for network broadcasts from a network device (e.g., access point) of communication network 200 and/or identifying the communication network to join based on a network name provided in the one or more configuration parameters 120. Alternatively, or in addition, the configuration steps may include connecting to the communication network 200 using a secure communication protocol based on network security information and/or password information provided in the one or more configuration parameters. Alternatively, or in addition, the configuration steps may include locating a server to communicate with via the communication network 200 based server information (e.g., a server address) provided in the one or more configuration parameters. In one example, the server and infusion endpoint(s) 112 may communicate access-controlled information via the network 200. In one embodiment, the server may be a management server and the access-controlled information may include information that may be transmitted to the infusion endpoint(s) 112. The infusion endpoint(s) may then perform a medical infusion procedure based, at least partially, on such access-controlled information.

The first communication medium may be disabled, for example, in response to the controller determining that the configuration parameters 120 for connecting to the communication network 200 and communicating with the server via the network 200 have been received, that a secure connection to the network 200 has been established, and/or that the infusion endpoint 112 can communicate with the server over the communication network 200. In some examples, the infusion endpoint 112 may not permit communication via the network link 216 until the first communication medium and wireless link 116 are disabled.

In the manner described above, each infusion endpoint 112 may be remotely, and in some examples, simultaneously configured. Thus, the infusion endpoint(s) 112 do not need to be individually configured by a technician, thereby facilitating a relatively quick and efficient network configuration and set up of the endpoints 112. Errors resulting from manual entry of configuration parameters may be reduced or avoided as well and configuration times may be reduced compared to individual, manual configuration of the infusion endpoints 112.

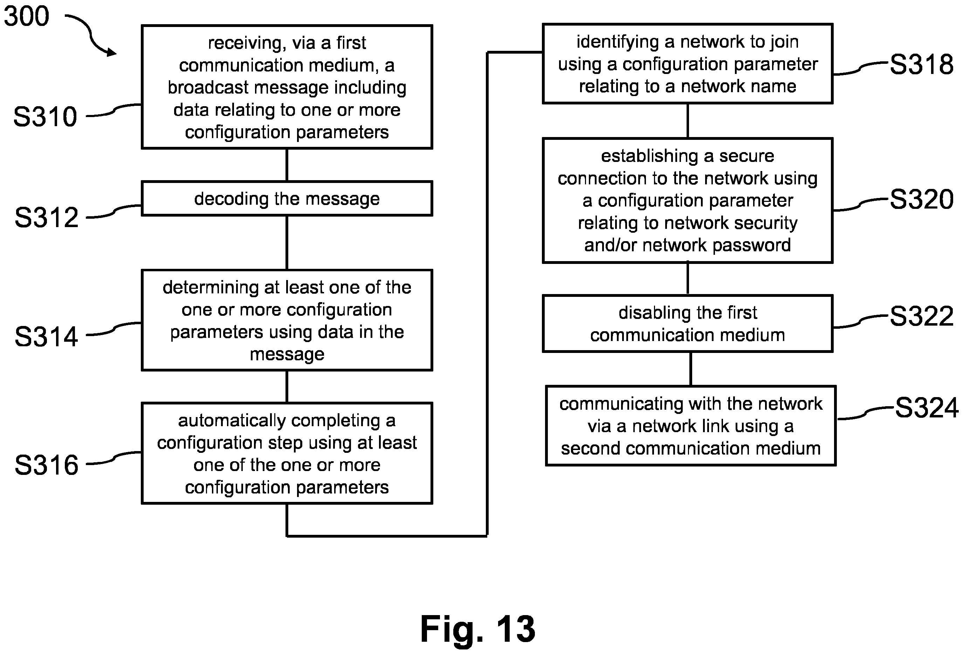

Fig. 13 is a block diagram showing an example of a method 300 for configuring one or more infusion endpoints 112 in an infusion system 100. An infusion endpoint 112 of the one or more infusion endpoints 112 includes a controller 134, a communication module 136, an endpoint user interface 138 and one or more pumps 144. At S310, the method includes receiving, by the communication module 136, via a wireless link 116 using a first communication medium, a broadcast message 118 including data relating to one or more configuration parameters 120. The message 118 may be encoded. At S312, the method optionally includes decoding, with a decoder 142, the encoded message 118. At S314, the method includes determining, by the controller 134, at least one of the one or more configuration parameters 120 using data in the message 118.

At S316, the method includes automatically completing, by the controller 134, a configuration step using at least one of the one or more configuration parameters 120. For example, at S318, the method may include identifying, by the controller 134, a network to join using a configuration parameter of the one or more configuration parameters relating to network name. Alternatively, or in addition, at S320, the method may include establishing a secure connection to the network using a configuration parameter relating to network security and/or a network password. At S322, the method may include disabling the first communication medium. At S324, the method may include communicating with the network via a network link 216 using a second communication medium. The second communication medium may be different from the first communication medium.

## Claims

1. An infusion system comprising:
an infusion endpoint configured to deliver a medical fluid, the infusion endpoint comprising:
a controller configured to control the infusion endpoint according to one or more configuration parameters; and
a communication module coupled to the controller configured to communicate using a first communication medium and a second communication medium; and
a broadcaster comprising a wireless transmitter in communication with the communication module via a wireless link using the first communication medium, and a computing device configured to provide a broadcasting utility,
wherein the broadcasting utility is configured to prepare a message that is transmitted by the wireless transmitter and received by the communication module, the message including data that is usable by the controller to determine at least one of the one or more configuration parameters,
wherein the controller is configured to control the infusion endpoint to connect to a communication network according to the one or more configuration parameters, wherein the communication module is configured to communicate with the communication network via a network link using the second communication medium, and
wherein the controller is configured to disable the first communication medium when the communication module is communicating using the second communication medium.

2. The infusion system according to claim 1, wherein the first communication medium is an audio frequency-shifted keyed medium or a Bluetooth protocol medium.

3. The infusion system according to claim 1 or 2, wherein the controller is configured to decode the message and determine the at least one of the one or more configuration parameters from the data.

4. The infusion system according to claim 3, wherein the controller is configured to automatically complete one or more configuration steps after the message has been decoded and the at least one of the one or more configuration parameters determined.

5. The infusion system according to any one of claims 1 to 4, comprising a plurality of infusion endpoints, and the broadcaster is configured to send the message to all of the infusion endpoints.

6. The infusion system according to any one of claims 1 to 4, comprising a plurality of infusion endpoints, and the broadcaster is configured to send the message to only one infusion endpoint of the plurality of infusion endpoints.

7. The infusion system according to any one of claims 1 to 6, wherein the infusion endpoint further comprises one or more pumps for delivering the medical fluid.

8. The infusion system according to claim 7, wherein the infusion endpoint is configured to receive one or more operating parameters from the communication network via the network link using the second communication medium.

9. The infusion system according to claim 8, wherein the controller is configured to control operation of the one or more pumps according to the one or more operating parameters.

10. The infusion system according to any one of claim 1 to 9, wherein the computing device is configured to receive the at least one of the one or more configuration parameters from one or more of an input device and a database server and provide the at least one of the one or more configuration parameters to the broadcasting utility.

11. The infusion system according to any one of claims 1 to 10, wherein the controller is coupled to an endpoint user interface and is configured to receive at least one other of the one or more configuration parameters from the endpoint user interface.

12. A method of configuring one or more infusion endpoints in an infusion system, each infusion endpoint comprising a controller, a communication module, one or more pumps for delivering a medical fluid, and an endpoint user interface, wherein the communication module is configured to communicate using a first communication medium and a second communication medium, the method comprising:
receiving, by the communication module, via a wireless link using the first communication medium, a broadcast message including data relating to one or more configuration parameters;
determining, by the controller, at least one of the one or more configuration parameters using the data in the message;
automatically completing, by the controller, a configuration step using at least one of the one or more configuration parameters, wherein the configuration step is a configuration step for connecting to a communication network;
establishing a secure connection, by the controller, with the communication network via a network link using the second communication medium; and
disabling, by the controller, communications using the first communication medium when the communication module communicates with the communication network using the second communication medium.

13. The method according to claim 12, wherein the message is an encoded message, and the method further comprises decoding the encoded message.

14. The method according to claim 12 or 13, wherein automatically completing the configuration step includes automatically identifying a network to join using a configuration parameter of the one or more configuration parameters relating to a network name, facility information and/or a network access point.

15. The method according to any one of claims 12 to 14, wherein automatically completing the configuration step includes automatically establishing the secure connection to the network using a configuration parameter relating to network security and/or a network password.
